# FASCICULE DE BREVET EUROPEEN

(11) **EP 4 342 524 B1**
(45) Date de publication et mention de la délivrance du brevet: **10.12.2025**
(21) Numéro de dépôt: 23191958.0
(22) Date de dépôt: 17.08.2023
(51) Int. Cl.: A61N 5/06

(54) **DISPOSITIF DE TRAITEMENT DE PHOTOTHÉRAPIE**
VORRICHTUNG ZUR PHOTOTHERAPIEBEHANDLUNG
PHOTOTHERAPY TREATMENT DEVICE

(30) Priorité: 24.09.2022 FR 2209702
(43) Date de publication de la demande: 27.03.2024
(73) Titulaire: Lucibel SA, 76360 Barentin (FR)
(72) Inventeur: GROS-DAILLON, Thibault, 76590 CRIQUETOT-SUR-LONGUEVILLE (FR); MOA, Yacine, 76510 Saint Nicolas d'Aliermont (FR)
(74) Mandataire: Martin, Marie-Aude

(56) Documents cités:
- CN-U- 208 185 983
- CN-U- 211 822 101
- CN-U- 213 642 864
- CN-U- 214 858 062
- US-A1- 2018 117 355

## Description

La présente invention concerne un dispositif destiné à fournir un traitement de photothérapie ou photo-modulation pour améliorer la santé de la peau, tel qu'un traitement anti-âge ou un traitement de prévention de l'acné, en utilisant plus particulièrement la photothérapie par diode électroluminescente (DEL).

Un tel traitement peut être réalisé à domicile au moyen d'un dispositif émetteur de lumière de photothérapie, réglable en hauteur et positionnable par exemple sur un bureau ou une table de chevet afin de permettre à l'utilisateur de se mettre en position assise ou bien allongée.

Toutefois, il est souhaitable de pouvoir proposer le traitement de photothérapie en institut spécialisé. Généralement, dans un tel institut, afin de favoriser sa relaxation et sa détente, l'utilisateur est installé dans une position allongée sur une banquette prévue à cet effet et une tête de traitement de l'appareil de traitement est approchée du corps allongé de l'utilisateur par différents réglages en hauteur et en orientation de l'appareil.

L'inconvénient des dispositifs de traitement par photothérapie de l'art antérieur est que l'ergonomie se fait généralement au détriment de l'esthétique. L'exposition au dispositif peut constituer une expérience intimidante et désagréable lorsque le traitement prend plusieurs minutes et que la tête de traitement est disposée relativement près du visage, provoquant souvent une sensation de claustrophobie et d'inconfort chez l'utilisateur au cours de la durée de traitement. Dans ce contexte, il est fait référence aux documents CN211822101 U et US2018/0117355 A1.

On connaît du document WO2006051985A1 un dispositif de photothérapie comprenant un émetteur de lumière de traitement de photothérapie en forme d'ovoïde supporté par un mât. L'émetteur de lumière rouge est raccordé par un tube flexible à une extrémité du mât. Le tube flexible est déformé en U pour permettre l'orientation de l'émetteur de lumière en direction du cuir chevelu d'un patient. Cette déformation risque de produire une usure prématurée du tube flexible qui est sollicité en torsion très régulièrement dans une zone coudée du tube. En outre, la déformation du tube flexible en U peut s'avérer fastidieuse pour l'opérateur en charge du positionnement de l'appareil.

L'invention a notamment pour but de fournir un appareil de photothérapie offrant une ergonomie et une esthétique améliorée par rapport aux appareils de l'art antérieur.

A cet effet, l'invention a pour objet un appareil de traitement par photothérapie d'une partie d'un corps d'un utilisateur, comprenant une tête de traitement comprenant une source lumineuse et un support pour maintenir la tête, dans lequel le support comprend un socle, un tube rigide raccordé au socle d'une part et à la tête d'autre part par l'intermédiaire d'un tube flexible, le tube flexible étant autoportant et pouvant se déformer selon une trajectoire curviligne tout en assurant une fonction de support de la tête afin de permettre la modification de l'orientation de la tête, caractérisé en ce que le tube rigide comprend un premier élément inférieur rectiligne et un deuxième élément supérieur, en forme de « J » inversé, avec une section rectiligne et une section curviligne et en ce que la section rectiligne du deuxième élément supérieur est montée de façon télescopique à l'intérieur du premier élément inférieur afin de permettre un réglage en hauteur de la tête par rapport au socle.

Grâce à l'invention, l'ergonomie de l'appareil de photothérapie est optimisée. D'une part une partie structurelle rigide permet un ajustement global et grossier de la hauteur de la tête et d'autre part une partie structurelle flexible permet une optimisation plus précise et affinée de l'orientation et de la hauteur.

En outre, l'emmanchement télescopique des deux éléments tubulaires inférieur et supérieur permet une intégration discrète de la mécanique de réglage de la hauteur de la tête.

Un appareil de photothérapie selon l'invention peut comprendre en outre l'une ou plusieurs des caractéristiques suivantes.

Dans un autre mode de réalisation de l'invention, la tête est un masque pourvu d'une armature délimitant une face frontale orientée en direction d'un visage d'un utilisateur et d'une face dorsale opposée, l'armature supportant une pluralité de points lumineux formant la source d'émission lumineuse, agencés dans le masque pour produire un éclairage énergétique du visage de l'utilisateur par la face frontale, dans une position d'utilisation.

Dans un autre mode de réalisation de l'invention, l'armature comprend une première paroi frontale intérieure destinée à être positionnée en regard du visage d'un utilisateur et une deuxième paroi dorsale extérieure conformée pour venir épouser la première paroi qui comprend un embout de raccordement au tube flexible, faisant saillie à partir d'une surface extérieure de la deuxième paroi.

Dans un autre mode de réalisation de l'invention, l'embout a une forme générale tronconique et comprend un canal traversant de réception d'une vis d'assemblage configurée pour coopérer par vissage à l'intérieur d'une première extrémité du tube flexible de raccordement au masque.

Dans un autre mode de réalisation de l'invention, l'embout comprend un insert métallique logé à l'intérieur du canal et pourvu d'un filetage interne femelle configuré pour coopérer avec la vis.

Dans un autre mode de réalisation de l'invention, les deux parois sont conformées pour présenter une concavité orientée en direction du visage à traiter.

Dans un autre mode de réalisation de l'invention, l'appareil comprend un circuit de raccordement électrique portant les points lumineux s'étendant entre les deux parois.

Dans un autre mode de réalisation de l'invention, l'armature comprend une plaque de dissipation thermique conformée pour venir épouser la concavité du corps et disposée entre les deux parois pour évacuer la chaleur susceptible d'être émise par la source lumineuse.

Dans un autre mode de réalisation de l'invention, le tube rigide supporte un boîtier de contrôle enfermant une carte électronique de contrôle de la source lumineuse de la tête selon un protocole de traitement prédéfini.

Dans un autre mode de réalisation de l'invention, l'appareil comprend un câble d'alimentation électrique de la source lumineuse destiné à s'étendre au travers des parties tubulaires du support entre le socle et la tête.

Dans un autre mode de réalisation de l'invention, le socle comprend une forme évasée qui s'élargit depuis une face supérieure raccordée au tube rigide vers une face inférieure supportant des roulettes.

Les dessins annexés illustrent l'invention :
[Fig.1] représente une vue en perspective d'un appareil de photothérapie selon l'invention ;
[Fig.2] représente une vue en perspective d'une tête de traitement de l'appareil de photothérapie et une partie de son support ;
[Fig.3] représente une vue partielle en perspective éclatée de la tête de la [Fig.2] ;
[Fig.4] représente une vue d'une face frontale du masque facial du dispositif de la [Fig.2] dans lequel une paroi intérieure est absente ;
[Fig.5] représente une vue extérieure en perspective d'une paroi extérieure du masque facial de la [Fig.2] ;
[Fig.6] représente une vue intérieure de face d'une paroi extérieure du masque facial de la [Fig.2] ;
[Fig.7] représente une vue en coupe de la paroi extérieure selon la ligne pointillée de la [Fig.6] ;
[Fig.8] représente une vue d'un tube flexible du support de maintien dans une configuration déployée longitudinalement.
[Fig.9] représente une vue de face d'une partie structurelle rigide du support de l'appareil de la [Fig.1].

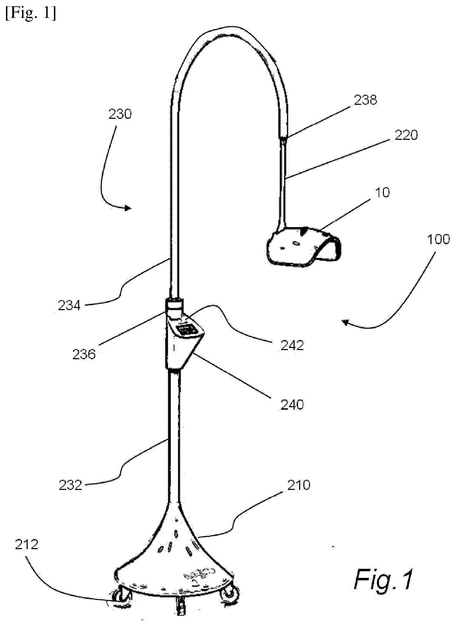

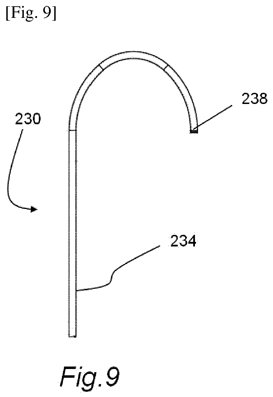

On a représenté sur les figures 1 à 9 un appareil de traitement par photothérapie selon l'invention. Cet appareil est désigné par la référence générale 100.

Plus particulièrement, l'appareil 100 comprend une tête de traitement 10 et un support 200 pour permettre le positionnement de la tête 10 comme cela sera détaillé ci-après.

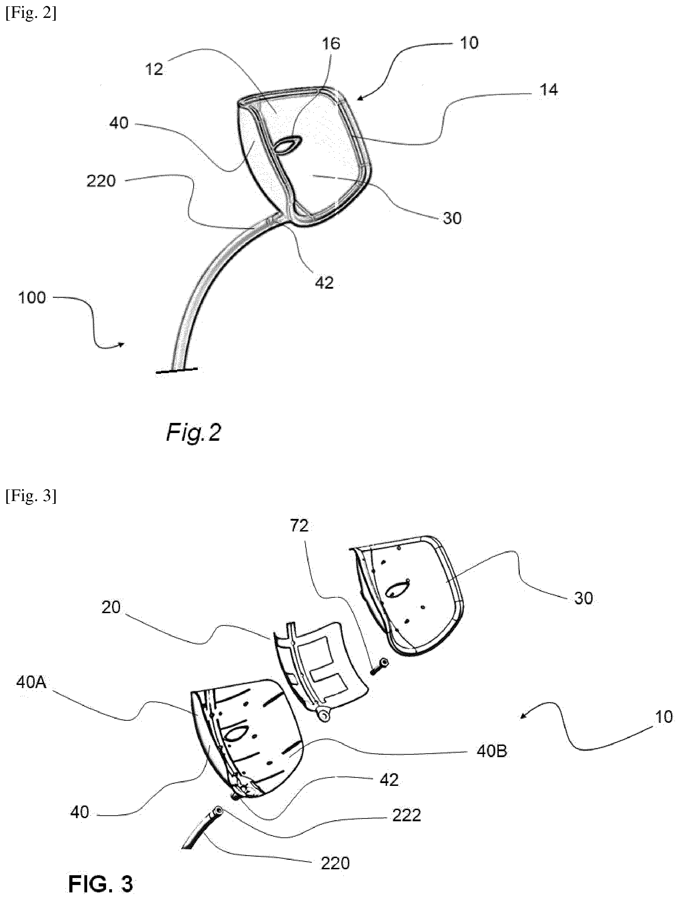

Comme cela est illustré en détail sur la [Fig.2], de préférence, la tête comprend un masque facial 10 comprenant une armature 12 relativement rigide de forme générale concave délimitée par un bord périphérique 14, destinée à être positionnée de préférence en regard d'une zone à traiter d'un utilisateur, ici le visage de l'utilisateur. Dans l'exemple décrit, l'armature 12 est formée d'une coque rigide conformée pour venir recouvrir l'ensemble du visage du patient dans une position d'utilisation.

En outre, l'armature 12 comprend encore une pluralité de points lumineux d'émission lumineuse 24 formant la source lumineuse 22. Dans ce premier mode de réalisation, les points lumineux 24 sont formés chacun par une diode électroluminescente avec un spectre de couleur rouge dans une longueur d'onde comprise principalement et de préférence entre 620 et 640 nm. De préférence, le flux lumineux émis par chaque diode électroluminescente 24 est au minimum de 30 lumens, par exemple compris dans la plage 30 lumens - 60 lumens.

Dans l'exemple illustré sur la [Fig.2], l'armature 12 est conformée pour couvrir le visage en épousant la courbure naturelle du visage et présente ainsi une concavité orientée en direction de la face de l'utilisateur. Cette concavité permet de recouvrir le visage d'un être humain en suivant la courbure naturelle entre les zones malaires d'une part et entre la zone frontale et la zone maxillaire inférieure d'autre part.

Dans cet exemple, la concavité du masque 10 permet notamment de mieux épouser et suivre les contours du visage d'un utilisateur et ainsi rendre plus efficace l'exposition au rayonnement lumineux émis par les points lumineux 22.

De préférence, comme cela est illustré sur la [Fig.3], l'armature 12 comprend une première paroi frontale intérieure 30 destinée à être positionnée en regard du visage de l'utilisateur. Cette paroi intérieure 30 est préformée et a une forme générale concave, par exemple curviligne pour s'adapter à la physionomie d'un visage humain. Ainsi, de préférence, comme cela est illustré en [Fig.3], la paroi intérieure 30 a une forme générale de masque facial et comprend une région centrale présentant au moins une courbure pour suivre la courbure naturelle d'un visage d'être humain.

La première paroi intérieure 30 est réalisée dans une matière laissant passer le flux lumineux de la source lumineuse 22. De préférence, la matière est translucide pour permettre une diffusion homogène de la lumière ainsi qu'une protection naturelle de l'utilisateur contre l'éblouissement. Par translucide, on entend un matériau « qui laisse passer la lumière mais qui n'est pas transparent ». Le choix d'un matériau translucide plutôt qu'un matériau transparent permet également au niveau esthétique de rendre moins visibles les points lumineux de la source lumineuse 22. Bien entendu, en variante, le matériau peut être choisi transparent aux rayons lumineux émis par les points lumineux 24 de la source lumineuse 22.

En outre, de préférence, la paroi intérieure 30 est réalisée dans une matière plastique thermoformable, par exemple un mélange de polystyrène et de polyéthylène (acronyme PS-PE).

La paroi intérieure 30 est ainsi de préférence formée à partir d'un matériau susceptible d'évoluer en fonction de la température entre une phase plastique et une phase rigide, par modelage dudit matériau dans sa phase plastique dans un moule, puis rigidification dudit matériau en place dans le moule.

En outre, conformément à l'invention, le dispositif 10 comprend une deuxième paroi 40 conformée pour venir épouser, dans sa configuration finale, la première paroi 30. Cette deuxième paroi 40 est également désignée par la suite par deuxième paroi dorsale extérieure 40.

Conformément à l'invention, les points lumineux 24 de la source lumineuse 22 sont intercalés entre les deux parois intérieure 30 et extérieure 40 formant l'armature 12.

Le procédé de fabrication générale de l'armature 12 comprend par exemple les étapes successives de (a) découpe d'une pièce dudit matériau, aux dimensions du moule selon une forme s'étalant dans le moule, et (b) mise en forme de ladite pièce, par modelage de la pièce de matériau dans sa phase plastique, (c) rigidification du matériau modelé, en place dans le moule pour former les parois de la coque rigide 12, et (d) aménagement d'évidements au niveau oculaire, soit sur la pièce avant sa mise en forme, soit une fois les parois 30, 40 de la coque rigidifiée.

De préférence, les parois 30 et 40 sont accouplées entre elles par des attaches, par exemple en matière plastique se présentant sous forme de clips (non représentés), et traversant des orifices coïncidant des deux parois 30 et 40. A cet effet, la paroi interne 30 présente une pluralité d'orifices et la paroi externe 40 comprend une pluralité d'orifices de fixation correspondants.

En outre, comme cela est illustré sur la [Fig.3], l'appareil 100 comprend un circuit 20 de raccordement électrique des sources lumineuses 24 à une source d'alimentation électrique. Dans cet exemple, le circuit 20 a une forme générale de plaque ajourée suffisamment déformable pour épouser la concavité du masque 10.

Sur la [Fig.1], on voit que le support 200 comprend un socle 210, une partie tubulaire rigide 230 raccordée au socle 210 d'une part et à la tête 10 d'autre part par l'intermédiaire d'une partie tubulaire flexible 220.

De préférence, la partie tubulaire flexible 220 est formée par un tube flexible autoportant permettant la modification de l'orientation de la tête 10 tout en la supportant et pouvant se déformer selon une trajectoire curviligne tout en assurant une fonction de support. Par exemple, la partie rigide 230 comprend un premier tube inférieur rectiligne 232 et un deuxième tube supérieur 234 partiellement curviligne en forme de « J » inversé. On comprendra que le deuxième tube supérieur 240 comprend une section rectiligne suivie d'une section curviligne formant par exemple un « U ».

En particulier, la section rectiligne de la deuxième tube supérieur 234 est montée de façon télescopique à l'intérieur du premier tube inférieur 232 afin d'ajuster la hauteur de la tête 10 par rapport au socle 210. Dans l'exemple décrit, les deux tubes 232 et 234 de la partie rigide 230 sont montés coulissants l'un dans l'autre, la section rectiligne du tube supérieur 234 formant un embout mâle dimensionné pour être emmanché à l'intérieur du tube inférieur 232 qui forme l'embout femelle récepteur.

Ainsi, la partie rigide 230 comprend deux tubes coulissants 232 et 234 l'un dans l'autre de sorte qu'un premier réglage en hauteur est obtenu par extension ou rétraction des deux tubes l'un dans l'autre.

En outre, de préférence, le support 200 comprend un moyen de blocage 236 permettant de verrouiller une position relative des deux tubes 232 et 234 l'un par rapport à l'autre dans une position souhaitée par un opérateur de l'appareil 100. Ce moyen de blocage 236 ne sera pas plus décrit et peut comprendre tout mécanisme adapté comme une bague de serrage, un collier de serrage, etc.

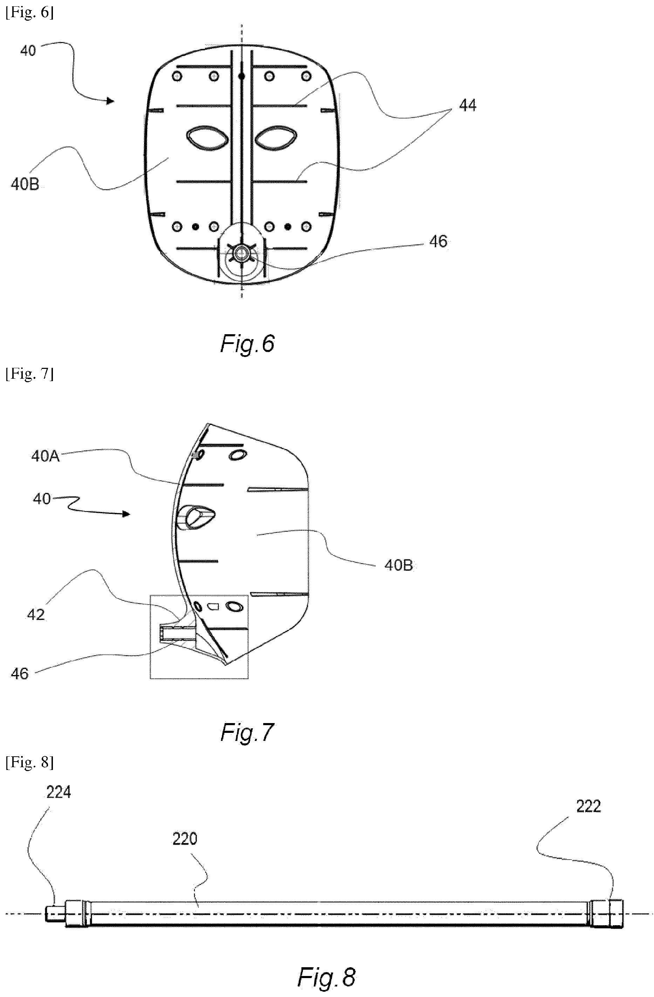

De préférence, le tube 234 est pourvu à une extrémité libre opposée à une extrémité coulissante à l'intérieur du tube 232, d'une platine 238 de fixation du tube flexible 220 au tube 234. Cette platine 238 comprend par exemple un taraudage prévu pour recevoir une extrémité 224 du tube flexible 220 ([Fig.8]) par exemple par vissage. La platine 238 est par exemple soudée à une extrémité du tube 234.

De préférence, le tube autoportant 220 permet de modifier également l'orientation du masque 10 de façon ergonomique. De préférence, le tube 220 flexible est configuré en « col de cygne » de façon à supporter le masque facial 10 tout en permettant de modifier également l'orientation du dispositif 10.

Un tube flexible dit « col de cygne » ou en anglais « stand tube » peut se déformer selon une trajectoire curviligne tout en assurant une fonction de support. De façon connue en soi, un tel tube est réalisé par exemple à partir d'une combinaison de deux enroulements généralement métalliques de sections ronde et triangulaire qui s'enroulent de façon alternée longitudinalement. On désignera par « âme métallique » du tube flexible 220, les enroulements du tube 220. Cette âme métallique est réalisée de préférence à partir d'un couple de matériaux métalliques comprenant de l'acier inoxydable et du laiton. Ce couple de matériau garantit une excellente résistance dans le temps sans générer de bruits indésirables liés au vieillissement.

De préférence, le support 200 comprend une gaine en matière plastique qui recouvre le tube flexible 220 sur toute sa longueur afin de permettre la manipulation du tube flexible 220 dans des conditions d'hygiène optimales.

De préférence, la gaine est montée dans une configuration dilatée autour de l'âme métallique du tube flexible 220, par exemple par gonflage de la gaine avec de l'air sous pression puis vient, par évacuation de l'air, épouser l'âme métallique du tube flexible 220 dans une configuration rétractée. Ainsi, la gaine dilatée par gonflage peut être aisément enfilée autour du tube flexible avant de se rétracter pour épouser étroitement l'âme métallique du tube flexible 220. Bien entendu, d'autres techniques de montage peuvent être utilisées telle qu'une technique de montage par thermo-rétraction de la gaine.

De préférence, le support 200 comprend au moins deux gaines superposées l'une sur l'autre autour du tube flexible 220. Un tel agencement présente l'avantage de masquer les enroulements métalliques ou âme métallique du tube flexible conférant au produit résultant un aspect de qualité accrue et une perception améliorée par l'utilisateur tout en offrant toujours autant de flexibilité au support.

Dans l'exemple illustré sur la [Fig.1], le tube rigide inférieur 232 supporte un boîtier 240 de contrôle enfermant une carte électronique de contrôle de la source lumineuse 22 selon un protocole de traitement prédéfini. En outre, de préférence, le boîtier 240 comprend une interface utilisateur 242. Comme cela est illustré sur la [Fig.1], le boîtier 240 a la forme générale d'un sabot s'étendant de préférence en extrémité du tube rigide 232 et présente une face supérieure élargie relativement plane sur laquelle s'étend l'interface utilisateur 242.

De préférence, l'appareil 100 comprend également un câble d'alimentation électrique de la source lumineuse 22 destiné à s'étendre entre le socle 210 et la source lumineuse 22 de la tête 10 en traversant le support 200. De préférence, comme cela est illustré sur la [Fig.1], le socle 210 comprend une forme évasée qui s'élargit depuis une face supérieure raccordée au tube rigide 232 vers une face inférieure supportant des roulettes 212. Cette forme évasée en entonnoir offre une grande stabilité de l'appareil tout en étant très esthétique.

Par exemple, le câble d'alimentation est de préférence destiné à être connecté à une de ses extrémités à une source d'alimentation électrique, par exemple l'alimentation générale d'un réseau électrique domestique et à l'autre de ses extrémités à la source lumineuse 22 logée à l'intérieur de la tête 10. Dans l'exemple décrit, le câble traverse le support 200 à l'intérieur des corps creux des tubes 232, 234 et 220.

A cet effet, de préférence, le circuit 20 comprend un bornier de raccordement à une extrémité du câble d'alimentation électrique. Dans l'exemple illustré, le bornier s'étend sur le tronc méridien 52, de préférence au voisinage d'une extrémité libre de ce tronc 52. De préférence, le bornier de raccordement électrique comprend un agencement de type presse-étoupe 70 au niveau de l'extrémité du câble pour assurer une étanchéité et une stabilité mécanique du circuit de sources lumineuses 22 vis-à-vis de l'environnement extérieur. De façon connue en soi, un agencement de type presse-étoupe 70 comprend une garniture compressible, à l'origine de l'étoupe qui est comprimée à l'aide d'un écrou ou d'une vis annulaire 72. Un tel presse-étoupe 70 permet de maintenir et d'assurer l'étanchéité autour du câble électrique. En outre, comme cela est visible en [Fig.3], la vis 72 permet également l'assemblage du masque 10 au support de maintien 200 comme cela sera décrit ultérieurement.

De préférence, le masque facial 10 comprend encore une interface de dissipation thermique (non illustrée sur les figures) réalisée par exemple en aluminium. Par exemple, cette interface de dissipation se présente sous la forme d'une plaque curviligne comprenant une ouverture pour le passage du câble d'alimentation et des évidements pour les yeux de l'utilisateur.

Comme cela est illustré sur la [Fig.8], le tube flexible 220 comprend une première extrémité de raccordement 222 au masque facial 10, de préférence par vissage au moyen de la vis d'assemblage 72. A cet effet, la première extrémité de raccordement 222 comprend une portion de filetage interne femelle propre à coopérer avec le filetage externe de la vis 72.

Dans le mode de réalisation préféré de l'invention, la vis 72, comme cela est visible sur la [Fig.3], est prévue pour assembler au moins le support de raccordement électrique 20, la paroi extérieure 40, et également de préférence la plaque de dissipation thermique, au tube flexible 220.

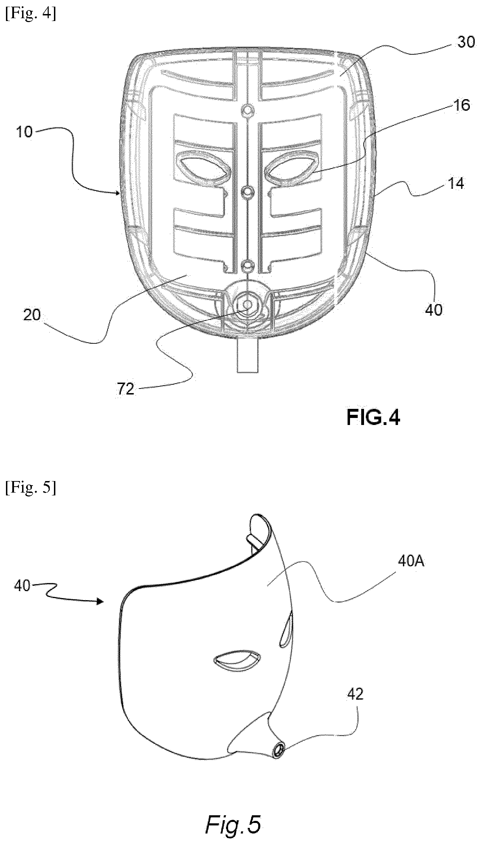

En outre, de préférence, la deuxième paroi extérieure 40 du masque facial 10 comprend un embout 42 de raccordement au tube flexible 220 faisant saillie à partir d'une surface externe 40A de la paroi 40. Cet embout 42 a une forme générale tronconique, en forme par exemple d'un bec creux, et délimite un canal tubulaire interne traversant la paroi extérieure 40. Comme cela est visible sur la [Fig.5], l'embout 42 se rétrécit par exemple en direction de son sommet à la manière d'un bec.

Ce canal est de préférence également pourvu d'un filetage interne femelle propre à coopérer avec le filetage de la vis 72 qui le traverse. A cet effet, cet embout 42 loge dans le canal, un insert métallique tubulaire 46 pourvu du filetage interne selon sa direction longitudinale. De préférence, cet insert métallique 46 est assemblé par surmoulage à la paroi externe 40 à l'intérieur de l'embout 42.

En outre, de préférence, la paroi extérieure 40 comprend des nervures de rigidification 44 sur sa face intérieure 40B.

Dans cet exemple, le tube flexible 220 comprend une deuxième extrémité 224 de raccordement au support. De préférence, comme cela est visible sur la [Fig.8], la deuxième extrémité 224 se présente sous la forme d'une tige filetée configurée pour coopérer avec la platine 238 par exemple par vissage ([Fig.1]).

De préférence, les première 222 et deuxième 224 extrémités de raccordement du tube flexible 220 sont formées par des pièces usinées puis serties à l'âme métallique du tube flexible 220.

On va maintenant décrire les principaux aspects du fonctionnement et de fabrication d'un appareil de traitement et dispositif de traitement selon l'invention.

En référence à la [Fig.1], lors de la mise sous tension de l'appareil 100, un opérateur règle grossièrement la hauteur du masque 10 en faisant coulisser le tube 234 à l'intérieur du tube 232 puis il ajuste plus finement le réglage grâce à la flexibilité du tube 220, la position du masque relativement au visage de manière à ce que ces yeux coïncident avec les évidements 16 du masque 10. Le protocole de traitement peut alors démarrer.

Bien entendu, l'invention ne se limite pas aux modes de réalisation précédemment décrits. D'autres modes de réalisation à la portée de l'homme du métier peuvent aussi être envisagés sans sortir du cadre de l'invention définie par les revendications ci-après.

## Revendications

1. Appareil (100) de traitement par photothérapie d'une partie d'un corps d'un utilisateur, comprenant une tête (10) de traitement comprenant une source lumineuse (22) et un support (200) pour maintenir la tête (10), dans lequel le support (200) comprend un socle (210), un tube rigide (230) raccordé au socle (210) d'une part et à la tête (10) d'autre part par l'intermédiaire d'un tube flexible (220), le tube flexible (220) étant autoportant et pouvant se déformer selon une trajectoire curviligne tout en assurant une fonction de support de la tête (10) afin de permettre la modification de l'orientation de la tête (10), dont le tube rigide (230) comprend un premier élément inférieur rectiligne (232) et un deuxième élément supérieur (234), en forme de « J » inversé, avec une section rectiligne et une section curviligne et en ce que la section rectiligne du deuxième élément supérieur (234) est montée de façon télescopique à l'intérieur du premier élément inférieur (232) afin de permettre un réglage en hauteur de la tête (10) par rapport au socle (210).

2. Appareil (100) selon la revendication précédente, dans lequel la tête (10) est un masque pourvu d'une armature (12) délimitant une face frontale orientée en direction d'un visage d'un utilisateur et d'une face dorsale opposée, l'armature (12) supportant une pluralité de points lumineux (24) formant la source d'émission lumineuse (22), agencés dans le masque (10) pour produire un éclairage énergétique du visage de l'utilisateur par la face frontale, dans une position d'utilisation.

3. Appareil (100) selon la revendication précédente, dans lequel l'armature (12) comprend une première paroi frontale intérieure (30) destinée à être positionnée en regard du visage d'un utilisateur et une deuxième paroi dorsale extérieure (40) conformée pour venir épouser la première paroi (30) qui comprend un embout (42) de raccordement au tube flexible (220), faisant saillie à partir d'une surface extérieure de la deuxième paroi (40).

4. Appareil (100) selon la revendication précédente, dans lequel l'embout (42) a une forme générale tronconique et comprend un canal traversant de réception d'une vis d'assemblage (72) configurée pour coopérer par vissage à l'intérieur d'une première extrémité (222) du tube flexible (220) de raccordement au masque (10).

5. Appareil (100) selon la revendication précédente, dans lequel l'embout (42) comprend un insert métallique (46) logé à l'intérieur du canal et pourvu d'un filetage interne femelle configuré pour coopérer avec la vis (72).

6. Appareil (100) selon l'une quelconque des revendications 2 à 5, dans lequel les deux parois (30, 40) sont conformées pour présenter une concavité orientée en direction du visage à traiter.

7. Appareil (100) selon l'une quelconque des revendications 2 à 6, comprenant un circuit (20) de raccordement électrique portant les points lumineux (22) s'étendant entre les deux parois (30, 40).

8. Appareil (100) selon l'une quelconque des revendications précédentes, dans lequel l'armature (12) comprend une plaque de dissipation thermique (60) conformée pour venir épouser la concavité du corps et disposée entre les deux parois (30, 40) pour évacuer la chaleur susceptible d'être émise par la source lumineuse (22).

9. Appareil (100) selon l'une quelconque des revendications précédentes, dans lequel le tube rigide (232) supporte un boîtier (240) de contrôle enfermant une carte électronique de contrôle de la source lumineuse (22) de la tête (10) selon un protocole de traitement prédéfini

10. Appareil (100) selon l'une quelconque des revendications précédentes, comprenant un câble d'alimentation électrique de la source lumineuse (22) destiné à s'étendre au travers des parties tubulaires du support (230) entre le socle (210) et la tête (10).

11. Appareil (100) selon l'une quelconque des revendications précédentes, dans lequel le socle (210) comprend une forme évasée qui s'élargit depuis une face supérieure raccordée au tube rigide (232) vers une face inférieure supportant des roulettes (212).

## Patentansprüche

1. Ein Phototherapie-Behandlungsgerät (100) zur Behandlung eines Körperteils eines Benutzers, umfassend einen Behandlungskopf (10) mit einer Lichtquelle (22) und einer Halterung (200) zur Befestigung des Behandlungskopfes (10), wobei die Halterung (200) umfasst:
- Einen Sockel (210),
- Ein starres Rohr (230), das einerseits mit dem Sockel (210) und andererseits über ein flexibles Rohr (220) mit dem Behandlungskopf (10) verbunden ist.
Das flexible Rohr (220) ist selbsttragend und kann sich entlang einer gekrümmten Bahn verformen, während es gleichzeitig den Behandlungskopf (10) stützt, um dessen Ausrichtung zu ermöglichen.
Das Gerät ist **dadurch gekennzeichnet, dass** das starre Rohr (230) umfasst:
- Ein erstes unteres gerades Element (232) und
- Ein zweites oberes Element (234), das die Form eines umgekehrten "J" hat, mit einem geraden Abschnitt und einem gekrümmten Abschnitt.
Der gerade Abschnitt des zweiten oberen Elements (234) ist teleskopisch innerhalb des ersten unteren Elements (232) montiert, um eine Höhenverstellung des Behandlungskopfes (10) relativ zum Sockel (210) zu ermöglichen.

2. Das Gerät (100) gemäß dem vorhergehenden Anspruch, wobei der Behandlungskopf (10) eine Maske ist, die mit einem Rahmen (12) ausgestattet ist, der eine Vorderseite in Richtung des Gesichts eines Benutzers und eine gegenüberliegende Rückseite definiert.
Der Rahmen (12) trägt eine Vielzahl von Lichtpunkten (24), die die Lichtquelle (22) bilden und innerhalb der Maske (10) so angeordnet sind, dass sie in der Gebrauchsposition das Gesicht des Benutzers mit energetischem Licht beleuchten.

3. Das Gerät (100) gemäß dem vorhergehenden Anspruch, wobei der Rahmen (12) umfasst:
- Eine erste innere vordere Wand (30), die zum Gesicht des Benutzers ausgerichtet ist, und
- Eine zweite äußere hintere Wand (40), die so geformt ist, dass sie sich an die erste Wand (30) anpasst.
Die zweite Wand (40) besitzt eine Anschlussdüse (42), die aus ihrer äußeren Oberfläche herausragt und zur Verbindung mit dem flexiblen Rohr (220) dient.

4. Das Gerät (100) gemäß dem vorhergehenden Anspruch, wobei die Anschlussdüse (42) eine trunkonische (kegelförmige) Form hat und einen Durchgangskanal umfasst, der eine Befestigungsschraube (72) aufnimmt, die durch Einschrauben mit dem ersten Ende (222) des flexiblen Rohrs (220) verbunden werden kann.

5. Das Gerät (100) gemäß dem vorhergehenden Anspruch, wobei die Anschlussdüse (42) ein Metalleinsatz (46) im Inneren des Kanals enthält, der mit einem weiblichen Innengewinde versehen ist, um mit der Schraube (72) zu interagieren.

6. Das Gerät (100) gemäß einem der Ansprüche 2 bis 5, wobei die beiden Wände (30, 40) so geformt sind, dass sie eine nach innen gewölbte konkave Form aufweisen, die auf das zu behandelnde Gesicht ausgerichtet ist.

7. Das Gerät (100) gemäß einem der Ansprüche 2 bis 6, umfassend einen elektrischen Anschlusskreis (20), der die Lichtpunkte (22) trägt und sich zwischen den beiden Wänden (30, 40) erstreckt.

8. Das Gerät (100) gemäß einem der vorhergehenden Ansprüche, wobei der Rahmen (12) eine Wärmeableitplatte (60) umfasst, die so geformt ist, dass sie sich der konkaven Körperform anpasst und zwischen den beiden Wänden (30, 40) angeordnet ist, um die von der Lichtquelle (22) erzeugte Wärme abzuleiten.

9. Das Gerät (100) gemäß einem der vorhergehenden Ansprüche, wobei das starre Rohr (232) ein Steuergehäuse (240) trägt, das eine elektronische Steuerplatine enthält, welche die Lichtquelle (22) im Behandlungskopf (10) gemäß einem vordefinierten Behandlungsprotokoll steuert.

10. Das Gerät (100) gemäß einem der vorhergehenden Ansprüche, umfassend ein Stromversorgungskabel für die Lichtquelle (22), das durch die tubulären Teile der Halterung (230) zwischen dem Sockel (210) und dem Behandlungskopf (10) verläuft.

11. Das Gerät (100) gemäß einem der vorhergehenden Ansprüche, wobei der Sockel (210) eine erweiterte Form hat, die sich von einer oberen Fläche, die mit dem starren Rohr (232) verbunden ist, zu einer unteren Fläche verbreitert, die mit Rollen (212) ausgestattet ist.

## Claims

1. A phototherapy treatment device (100) for a part of a user's body, comprising a treatment head (10) that includes a light source (22) and a support structure (200) to hold the treatment head (10), wherein the support structure (200) comprises a base (210), a rigid tube (230) connected on one end to the base (210) and on the other end to the treatment head (10) via a flexible tube (220). The flexible tube (220) is self-supporting, capable of bending along a curved path while still supporting the treatment head (10), allowing for adjustment of the head's orientation. The device is **characterized in that** the rigid tube (230) comprises:
- A first lower straight element (232), and
- A second upper element (234), shaped like an inverted "J", with both a straight section and a curved section.
Additionally, the straight section of the second upper element (234) is telescopically mounted inside the first lower element (232), allowing for height adjustment of the treatment head (10) relative to the base (210).

2. The device (100) according to the previous claim, wherein the treatment head (10) is a mask with a frame (12) that defines a front face directed towards a user's face and an opposite rear face. The frame (12) supports multiple light-emitting points (24) forming the light source (22), arranged in the mask (10) to provide energy-based illumination to the user's face when in use.

3. The device (100) according to the previous claim, wherein the frame (12) comprises:
- A first inner front wall (30) positioned towards the user's face, and
- A second outer rear wall (40), designed to conform to the first wall (30).
The second wall (40) includes a connector nozzle (42) protruding from its exterior surface, which attaches to the flexible tube (220).

4. The device (100) according to the previous claim, wherein the connector nozzle (42) has a truncated cone shape and includes a through-channel designed to accommodate an assembly screw (72), which can be screwed into the first end (222) of the flexible tube (220) for connection to the mask (10).

5. The device (100) according to the previous claim, wherein the connector nozzle (42) contains a metal insert (46) inside the channel, equipped with an internal female thread designed to fit the screw (72).

6. The device (100) according to any of claims 2 to 5, wherein the two walls (30, 40) are shaped to have a concavity directed towards the face to be treated.

7. The device (100) according to any of claims 2 to 6, comprising an electrical connection circuit (20) that carries the light-emitting points (22), extending between the two walls (30, 40).

8. The device (100) according to any of the preceding claims, wherein the frame (12) includes a heat dissipation plate (60) shaped to conform to the body's concavity and positioned between the two walls (30, 40) to dissipate heat generated by the light source (22).

9. The device (100) according to any of the preceding claims, wherein the rigid tube (232) supports a control box (240) containing an electronic control board for managing the light source (22) in the treatment head (10) based on a predefined treatment protocol.

10. The device (100) according to any of the preceding claims, comprising an electrical power cable for the light source (22), extending through the tubular parts of the support structure (230) between the base (210) and the treatment head (10).

11. The device (100) according to any of the preceding claims, wherein the base (210) has a flared shape, widening from an upper surface connected to the rigid tube (232) to a lower surface fitted with wheels (212).
